# EUROPEAN PATENT SPECIFICATION

(11) **EP 2 995 294 B1**
(45) Date of publication and mention of the grant of the patent: **08.01.2020**
(21) Application number: 14794702.2
(22) Date of filing: 09.05.2014
(51) Int. Cl.: A61K 8/63, A61K 8/19, A61K 8/362, A61Q 19/10, A61K 8/97

(54) **BATHING AGENT COMPOSITION**
BADEMITTELZUSAMMENSETZUNG
COMPOSITION D'AGENT DE BAIN

(30) Priority: 10.05.2013 JP 2013099817
(43) Date of publication of application: 16.03.2016
(73) Proprietor: Kao Corporation, Tokyo 103-8210 (JP)
(72) Inventor: HORI, Takaaki, Tokyo 131-8501 (JP); HORIUCHI, Arisa, Tokyo 131-8501 (JP); SUGIYAMA, Mitsuru, Haga-gun Tochigi 321-3497 (JP); NOMURA, Tomoko, Haga-gun Tochigi 321-3497 (JP)
(74) Representative: Hoffmann Eitle
(86) International application number: PCT/JP2014/062430
(87) International publication number: WO 2014/181852

(56) References cited:
- JP-A- S60 215 618
- JP-A- 2003 160 497
- JP-A- 2003 286 155
- KR-A- 20100 079 416
- KR-B1- 100 706 111
- DATABASE GNPD [Online] MINTEL; September 2005 (2005-09), "Rice Extract Detoxifying Bath Soak", XP002764548, Database accession no. 10231753
- DATABASE GNPD [Online] MINTEL; December 2001 (2001-12), "Relaxing Bath Tablets", XP002764549, Database accession no. 126544
- POPOVICH D G ET AL: "Generation of ginsenosides Rg3 and Rh2 from North American ginseng", PHYTOCHEMI, PERGAMON PRESS, GB, vol. 65, no. 3, 1 February 2004 (2004-02-01), pages 337-344, XP004485981, ISSN: 0031-9422, DOI: 10.1016/J.PHYTOCHEM.2003.11.020

## Description

### Field of the Invention

The present invention relates to a bath agent composition and use of the bath agent composition for whole-body bathing or for foot bathing.

### Background of the Invention

Heretofore, a bath agent obtained by blending a carbonate and an organic acid has been widely known as an excellent bath agent because the agent generates bubbles of carbon dioxide gas in bath water, and the carbon dioxide gas provides an effect of promoting blood circulation. In addition, investigations for providing an additional effect or a synergistic effect by further blending various components in the bath agent have been made.

For example, a crude drug such as Asian ginseng has been known as a component capable of being blended in a so-called foaming bath agent obtained by blending a carbonate and an organic acid such as succinic acid, and a foaming bath agent that includes such component and thus can synergistically enhance an effect of promoting blood circulation and moist feeling of skin after bathing has been developed (see Patent Document 1).

Patent Document 2 describes a bath agent composition comprising:
red ginseng residue as a main ingredient produced by mixing 50 to 80 parts by weight of dried red ginseng residue which is dry-treated to have a moisture content of 5 to 15% by weight and pulverized to a size of 10 to 100 mesh;
0.05 to 2 parts by weight of medicinal herb extracts extracted from a mixture of Angelica gigas Nakai, Chestnut inner shell, Ganoderma lucidum, Bamboo, Dandelion, Saururus chinensis (Lour.) Baill, Artemisia capillaris Thunberg, white ginseng, and red ginseng;
6 to 20 parts by weight of sodium sulfate;
4 to 15 parts by weight of citric acid;
4 to 15 parts by weight of sun-dried sea salt;
2 to 10 parts by weight of sodium hydrogen carbonate; and
0.4 to 4 parts by weight of sodium carbonate.

Asian ginseng is also referred to as *Panax ginseng,* and is a medicinal plant known from old times. Ginsenoside is known as its medicinal ingredient, and 50 kinds or more of ginsenosides have hitherto been isolated from *Panax ginseng.* In recent years, as effects of *Panax ginseng* or ginsenosides, a wide variety of effects such as an anti-tumor action, an anti-arteriosclerotic/anti-hypertensive action, an anti-stress action, an immunomodulating action, an anti-inflammatory/anti-allergic action, an anti-diabetic action, an action on a central nerve, an action of enhancing memory or learning, an action of protecting nerves, and an action of releasing or taking in neurotransmitters have been found, in addition to a well-known tonic action (Non Patent Literatures 1 and 2).

[Patent Document 1] JP-A-60-215618
[Patent Document 2] KR 2010-0079416 A

[Non Patent Document 1] Advances in Food and Nutrition Research, 2009, 55: 1-99
[Non Patent Document 2] Phytochemical Analysis, 2008, 19: 2-16

### Summary of the Invention

The present invention provides a bath agent composition, comprising the following components (A), (B), and (C):
(A) a total of 0.00001 mass% or more and 0.5 mass% or less of ginsenoside Rg3 and/or ginsenoside Rh2;
(B) a carbonate comprising 30 mass% or more of a dialkali metal carbonate; and
(C) an organic acid,
wherein a mass ratio of ginsenosides Rb2, Rb1, Rc, and Rd to ginsenosides Rg3 and Rh2 ((Rb2+Rb1+Rc+Rd)/(Rg3+Rh2)) is 0 or more and 30 or less.

The present invention also relates to the use of the bath agent composition for whole-body bathing or for foot bathing.

A conventional bath agent which comprises a crude drug such as *Panax ginseng,* a carbonate, and an organic acid and is obtained by merely blending these components is likely to be colored in itself. In addition, the conventional bath agent has risks of being discolored with time and reduced in appearance stability as well. In addition, the conventional bath agent still leaves room to investigate improvement of skin feeling at the time of bathing, and is likely not to give the additional effect or synergistic effect to be expected sufficiently.

Accordingly, the present invention relates to a bath agent composition capable of sufficiently exhibiting an effect of promoting blood circulation obtained by blending a carbonate and an organic acid, enhancing appearance stability, suppressing coloration and discoloration, and further, improving skin feeling at the time of bathing.

In view of the foregoing, the inventors of the present invention made various investigations and found that, when a bath agent composition comprises specific two kinds of ginsenosides at a specific mass ratio among ginsenosides known to be mainly contained in *Panax ginseng* and a specific carbonate, the bath agent composition can realize enhancement in appearance stability while synergistically enhancing an effect of promoting blood circulation obtained by generation of carbon dioxide gas, and provide satisfactory skin feeling at the time of bathing. Thus, the present invention was completed.

According to the bath agent composition of the present invention, it is possible to synergistically enhance the effect of promoting blood circulation in combination with the carbonate and the organic acid, and further, enhance the appearance stability and effectively suppress the coloration of the bath agent composition and the discoloration with time of the composition. In addition, it is possible to make the skin feeling at the time of bathing moist, highly oily, and smooth one.

### Detailed Description of the Invention

Now, the present invention is described in detail.

A bath agent composition of the present invention comprises a total of 0.00001 mass% or more and 0.5 mass% or less of ginsenoside Rg3 and/or ginsenoside Rh2 as a component (A). Ginsenoside Rg3 and ginsenoside Rh2 are also known as active ingredients of *Panax ginseng.* Ginsenoside Rg3 is represented by the following formula (1), and ginsenoside Rh2 is represented by the following formula (2) .

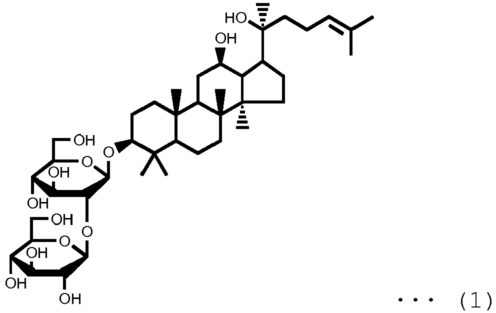

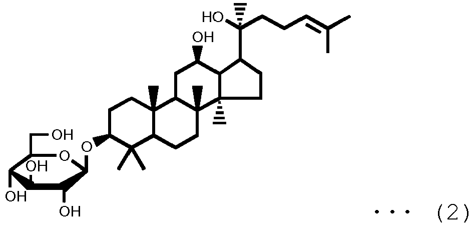

Ginsenoside Rg3 and ginsenoside Rh2 of the component (A) each can effectively act and exhibit an effect of promoting blood circulation, and further can enhance the effect of promoting blood circulation along with a carbonate of a component (B) and an organic acid of a component (C) described below through generation of carbon dioxide gas. In addition, appearance stability can be enhanced, and not only coloration of the bath agent composition but also discoloration with time of the composition can be effectively suppressed. Further, moist, highly oily and smooth skin feeling at the time of bathing can be achieved, that is, when touching the skin in bath water, such slimy feeling as to be coated with a thin oil film can be moderately sensed, and frictional feeling such as squeaky feeling can be suppressed. One of ginsenoside Rg3 and ginsenoside Rh2 may be used alone, or both of ginsenoside Rg3 and ginsenoside Rh2 may be used in combination. Of those, ginsenoside Rg3 is preferred from the viewpoints of synergistically enhancing the effect of promoting blood circulation, and enhancing the effect of suppressing coloration and discoloration.

A content of the component (A) in the bath agent composition of the present invention is 0.00001 mass% or more, preferably 0.00002 mass% or more, more preferably 0.00003 mass% or more in total, from the viewpoints of enhancing the effect of promoting blood circulation, the effect of providing satisfactory skin feeling at the time of bathing, and the effect of suppressing coloration and discoloration. The content of the component (A) in the bath agent composition of the present invention is 0.5 mass% or less, preferably 0.3 mass% or less, more preferably 0.1 mass% or less in total, from the viewpoints of ensuring the generation of carbon dioxide gas, and satisfactorily maintaining the effect of suppressing coloration and discoloration. In addition, the content of the component (A) in the bath agent composition of the present invention is from 0.00001 mass% to 0.5 mass%, preferably from 0.00002 mass% to 0.3 mass%, more preferably from 0.00003 mass% to 0.1 mass% in total.

Ginsenosides Rb2, Rb1, Rc and Rd are also known as components that may be contained in *Panax ginseng* together with ginsenosides Rg3 and Rh2 of the component (A). In the bath agent composition of the present invention, a mass ratio of ginsenosides Rb2, Rb1, Rc and Rd to ginsenosides Rg3 and Rh2 of the component (A) ((Rb2+Rb1+Rc+Rd)/(Rg3+Rh2)) is 0 or more from the viewpoint of enhancing the effect of suppressing coloration and discoloration, and is 30 or less, preferably 20 or less, more preferably 10 or less, more preferably 7 or less, more preferably 3 or less, even more preferably 1 or less, from the viewpoint of satisfactorily maintaining the effect of suppressing coloration and discoloration. The mass ratio of ginsenosides Rb2, Rb1, Rc and Rd to ginsenosides Rg3 and Rh2 of the component (A) ((Rb2+Rb1+Rc+Rd)/(Rg3+Rh2)) is from 0 to 30, preferably from 0 to 20, more preferably from 0 to 10, more preferably from 0 to 7, more preferably from 0 to 3, even more preferably from 0 to 1.

Ginsenosides Rb2, Rb1, Rc and Rd are represented by the following formulae (3), (4), (5), and (6), respectively.

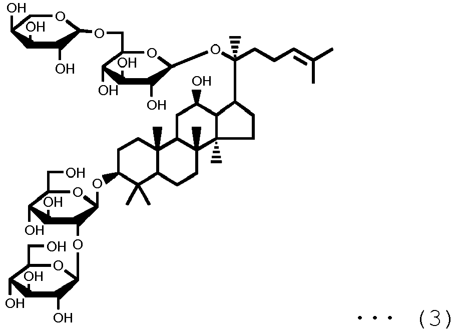

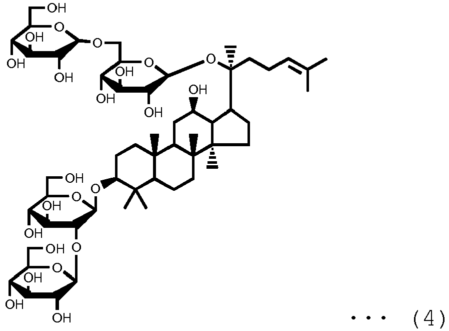

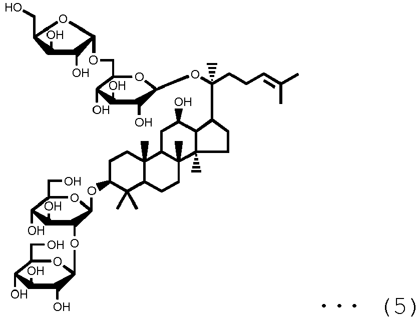

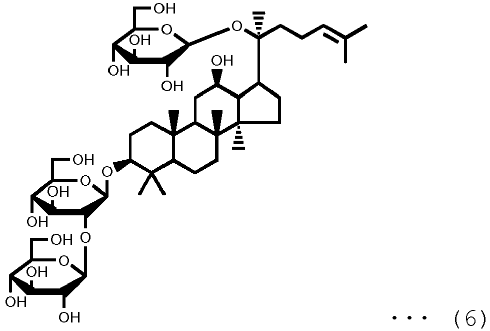

For example, in the case of extracting ginsenoside from *Panax ginseng,* a plurality of kinds of ginsenosides, not just ginsenosides Rg3 and Rh2, are generally extracted in a mixed form. However, in the present invention, attention is focused on ginsenosides Rg3 and Rh2, and the content thereof is set to the specific content and the ratio (Rb2+Rb1+Rc+Rd)/(Rg3+Rh2) is set to fall within the above-mentioned range. With this, the effect of promoting blood circulation, the effect of suppressing coloration and discoloration and the effect of providing satisfactory skin feeling at the time of bathing by virtue of ginsenosides Rg3 and Rh2 can be sufficiently exhibited.

Furthermore, for example, in the case of obtaining the component (A) by extracting ginsenoside from *Panax ginseng* such the ratio (Rb2+Rb1+Rc+Rd)/(Rg3+Rh2) falls within the above-mentioned range, a method of extracting under high temperature and high pressure, a method of extracting using an enzyme, a method of extracting using an acid such as vinegar or a mineral acid, a method of extracting using an ion-exchange resin, or the like may be adopted. Specifically, for example, the extraction is performed by using the root of *Panax ginseng* as a raw material and water, ethanol, 2-propanol, butanol, or a mixed solvent thereof as an extraction solvent, at a temperature of from 60°C to 100°C over a time period of from 0.5 hour to 40 hours. It is preferred that the extraction solvent be water, the extraction temperature be from 90°C to 100°C, and the extraction time period be from 20 hours to 40 hours. The extract obtained by the extraction is subjected to column chromatography using, e.g., an aromatic column such as DIAION HP20, or a HPLC ODS column to separate ginsenosides for isolation. Thus, the ratio (Rb2+Rb1+Rc+Rd)/(Rg3+Rh2) can be adjusted to fall within the above-mentioned range. Further, in addition to *Panax ginseng, Panax notoginseng, Panax bipinnatifidus, Panax japonicus, Panax quinquefolius, Panax vietnamensis, Panax wangianus, Panax zingiberensis, Panax pseudoginseng, Panax stipuleanatus, and Panax trifolius* may also be subjected to extraction so that the ratio (Rb2+Rb1+Rc+Rd)/(Rg3+Rh2) may fall within the above-mentioned range.

The bath agent composition of the present invention comprises a carbonate comprising 30 mass% or more of a dialkali metal carbonate, as a component (B). When the bath agent composition of the present invention is put into bath water, the component (B) generates carbon dioxide gas together with the organic acid of the component (C) described below, and thus can provide the effect of promoting blood circulation and impart satisfactory warm feeling to skin. As the dialkali metal carbonate, one kind or two kinds selected from sodium carbonate and potassium carbonate are mentioned. Of those, sodium carbonate is preferred from the viewpoint of ensuring high preservation stability.

A content of the dialkali metal carbonate in the carbonate of the component (B) is 30 mass% or more, preferably 40 mass% or more, more preferably 50 mass% or more, from the viewpoints of ensuring the appearance stability and enhancing the effect of suppressing coloration and discoloration. The content of the dialkali metal carbonate in the carbonate of the component (B) is preferably 100 mass% or less, more preferably 99 mass% or less, even more preferably 95 mass% or less, from the viewpoints of ensuring the appearance stability, maintaining a satisfactory effect of suppressing coloration and discoloration, and achieving an increase in solubility in bath water. In addition, the content of the dialkali metal carbonate in the carbonate (B) is 30 mass% or more, preferably from 30 mass% to 100 mass%, more preferably from 40 mass% to 100 mass%, more preferably from 40 mass% to 99 mass%, even more preferably from 50 mass% to 95 mass%.

The bath agent composition of the present invention may comprise a carbonate other than the dialkali metal carbonate, that is, one or more kinds selected from a monoalkali metal carbonate such as sodium hydrogen carbonate or potassium hydrogen carbonate, and a carbonate of a divalent or higher-valent metal such as calcium carbonate in the component (B). Of those, as the carbonate other than the dialkali metal carbonate, a monoalkali metal carbonate is preferred, and one kind or two kinds selected from sodium hydrogen carbonate and potassium hydrogen carbonate are more preferred from the viewpoint of ensuring an amount of carbon dioxide gas to be generated. From the viewpoints of ensuring the amount of carbon dioxide gas to be generated and ensuring the appearance stability, a content of the monoalkali metal carbonate in the component (B) is preferably 60 mass% or less, more preferably 50 mass% or less.

A content of the carbonate of the component (B) in the bath agent composition of the present invention is preferably 20 mass% or more, more preferably 25 mass% or more, even more preferably 30 mass% or more, from the viewpoints of ensuring the amount of carbon dioxide gas to be generated to provide a satisfactory effect of promoting blood circulation, and enhancing the effect of suppressing coloration and discoloration. The content of the carbonate of the component (B) in the bath agent composition of the present invention is preferably 70 mass% or less, more preferably 65 mass% or less, even more preferably 60 mass% or less, from the viewpoints of ensuring the amount of carbon dioxide gas to be generated, providing a sufficient effect of promoting blood circulation, and satisfactorily maintaining the effect of suppressing coloration and discoloration. In addition, the content of the carbonate of the component (B) in the bath agent composition of the present invention is preferably from 20 mass% to 70 mass%, more preferably from 25 mass% to 65 mass%, even more preferably from 30 mass% to 60 mass%.

In addition, a mass ratio of ginsenosides Rg3 and Rh2 of the component (A) to the component (B), ((Rg3+Rh2)/(B)), is preferably 0.0005×10⁻³ or more, more preferably 0.0025×10⁻³ or more, even more preferably 0.02×10⁻³ or more, from the viewpoint of concurrently enhancing the effect of promoting blood circulation and the effect of suppressing coloration and discoloration. The mass ratio of ginsenosides Rg3 and Rh2 of the component (A) to the component (B), ((Rg3+Rh2)/(B)), is preferably 3×10⁻³ or less, more preferably 2.5×10⁻³ or less, even more preferably 2×10⁻³ or less, from the viewpoint of maintaining the satisfactory effect of promoting blood circulation and the effect of suppressing coloration and discoloration in a balanced state. The mass ratio of ginsenosides Rg3 and Rh2 of the component (A) to the component (B), ((Rg3+Rh2)/(B)), is preferably from 0.0005×10⁻³ to 3×10⁻³, more preferably from 0.0025×10⁻³ to 2.5×10⁻³, even more preferably from 0.02×10⁻³ to 2×10⁻³.

The bath agent composition of the present invention comprises an organic acid of a component (C). The component (C) is preferably an organic acid that is solid at room temperature (25°C) such as malic acid, tartaric acid, citric acid, maleic acid, succinic acid, phthalic acid, fumaric acid, glutaric acid, adipic acid, benzoic acid, salicylic acid, and oxalic acid. One kind of those may be used alone, or two or more kinds thereof may be used in combination. Of those, one kind or more kinds selected from citric acid, malic acid, succinic acid, and fumaric acid are preferred from the viewpoint of ensuring the appearance stability.

A content of the component (C) in the bath agent composition of the present invention is preferably 20 mass% or more, more preferably 25 mass% or more, even more preferably 30 mass% or more, from the viewpoints of ensuring the amount of carbon dioxide gas to be generated to provide a satisfactory effect of promoting blood circulation, and enhancing the effect of suppressing coloration and discoloration. The content of the component (C) in the bath agent composition of the present invention is preferably 70 mass% or less, more preferably 65 mass% or less, even more preferably 60 mass% or less, from the viewpoints of ensuring the amount of carbon dioxide gas to be generated to provide a satisfactory effect of promoting blood circulation, and satisfactorily maintaining the effect of suppressing coloration and discoloration. In addition, the content of the component (C) in the bath agent composition of the present invention is preferably from 20 mass% to 70 mass%, more preferably from 25 mass% to 65 mass%, even more preferably from 30 mass% to 60 mass%.

The bath agent composition of the present invention may further comprise a dye as a component (D). For example, a content of the dye of the component (D) may be 0.0001 mass% or more and 5.0 mass% or less, and is preferably 0.5 mass% or less, more preferably 0.05 mass% or less. In addition, the bath agent composition of the present invention is preferably free of the dye of the component (D). The bath agent composition of the present invention, which comprises the specific amount of the component (A), the component (B) and the component (C), and has the ratio (Rb2+Rb1+Rc+Rd)/(Rg3+Rh2) falling within the specific range, can exhibit high appearance stability and effectively suppressed coloration and discoloration with time, and hence can achieve a reduction in content of the dye of the component (D), or eliminate the need for the dye of the component (D). Thus, a satisfactory color tone can be maintained for a long time period.

The meaning of dye of the component (D) encompasses a pigment as well, and specific examples of such component (D) include: tar dyes such as Blue No. 1, Red No. 106, Red No. 2, Yellow No. 4, Yellow No. 202(1), and Green No. 3, which are defined by tar dyes specified by the ordinance of the Ministry of Health, Labour and Welfare in the Appended Tables I and II; natural dyes such as chlorophyll, riboflavin, and annatto, which are approved as food additives; and pigments such as titanium oxide, zinc oxide, talc, kaolin, bentonite, and titanated mica. One kind of those dyes may be used alone, or two or more kinds thereof may be used in combination.

The bath agent composition of the present invention may preferably comprise a water-soluble polymer as a component (E) from the viewpoint of increasing the dispersibility of the components described above at the time of putting the composition into bath water. Examples of such component (E) include: natural water-soluble polymers such as glue, gelatin, collagen protein, casein, sodium alginate, carrageenan, furcellaran, tamarind gum, pectin, gum arabic, guar gum, xanthan gum, gum tragacanth, locust bean gum, dextrin, dextran, agar, and starch; semisynthetic water-soluble polymers such as carboxymethylcellulose, methylcellulose, hydroxyethylcellulose, hydroxypropylcellulose, cellulose acetate phthalate, alginic acid propylene glycol ester, oxidized starch, esterified starch, etherified starch, and cationic starch; and synthetic water-soluble polymers such as sodium polyacrylate, polyethylene imine, polyvinyl alcohol, polyethylene glycol, and polyvinylpyrrolidone. One kind of those polymers may be used alone, or two or more kinds thereof may be used in combination. Of those, one kind or two kinds selected from polyethylene glycol and dextrin are preferred, and polyethylene glycol is more preferred.

In addition, it is preferred that the component other than the component (B) be coated with the component (E) from the viewpoint of enhancing the effect of suppressing coloration and discoloration. The component other than the component (B) is preferably the organic acid of the component (C) from the viewpoints of ensuring separability from the component (B) to increase the amount of carbon dioxide gas to be generated, and maintaining satisfactory preservation stability. As the component (E) for coating the component other than the component (B), polyethylene glycol is preferably used. A mass ratio of the component other than the component (B) to be coated to the component (E) for coating the component other than the component (B), (the component to be coated/the component (E) for coating), is preferably from 4 to 90, more preferably from 6 to 40. In order to coat the component other than the component (B) with the component (E), it is preferred that these components be mixed and then granulated with an extrusion granulator, a tumbling granulator, or the like.

Furthermore, in the case of using the component other than the component (B) coated with the component (E), the bath agent composition of the present invention may further comprise the component (E) as another component. Of the component (E), the component (E) for coating the component other than the component (B) and the component (E) contained as another component in the bath agent composition of the present invention may be the same as or different from each other.

A content of the component (E) in the bath agent composition of the present invention is preferably 0.5 mass% or more, more preferably 1 mass% or more, more preferably 3 mass% or more, even more preferably 5 mass% or more in total, from the viewpoint of increasing the dispersibility of the components. The content of the component (E) in the bath agent composition of the present invention is preferably 30 mass% or less, more preferably 25 mass% or less in total, from the viewpoint of ensuring the amount of carbon dioxide gas to be generated.

The bath agent composition of the present invention may appropriately comprise, as a component other than the above-mentioned components, a component that is usually used for a bath agent, for example, a non-ionic surfactant, an inorganic salt other than the carbonate, a vitamin, a protease, a bactericidal and antiseptic agent, a diluting agent, a flavor, a crude drug, or the like. Of those, the bath agent composition of the present invention preferably comprises silicic anhydride or the like having liquid absorbability, or sodium sulfate or the like capable of retaining water as water of crystallization, from the viewpoint of enhancing a satisfactory effect of suppressing coloration and discoloration.

The bath agent composition of the present invention may be produced by mixing components well and subjecting the mixture to a conventional method such as a compression granulation method such as extrusion granulation or a compression molding method using a compression tableting machine or a briquetting machine. The form of the composition may be any of powder, granule, particle, briquette tablet, tablet, and the like. In addition, the composition may have a form obtained by granulating or molding part of the components in advance, mixing the resultant product with the residual components, and molding the mixture. For example, in the case of coating the component other than the component (B) with the component (E), it is appropriate that these components be mixed in advance and granulated, and then the resultant product is mixed with the residual components.

The bath agent composition of the present invention is used as follows: the composition is dissolved in bath water to generate bubbles of carbon dioxide gas, and at least part of the body is soaked in the bath water. The body part to be soaked may be the whole body (whole-body bathing) or only part of the body such as the neck, shoulder, hand, arm, foot, or leg. Of those, the bath agent composition of the present invention is an ideal foot bath agent to be used as soaking the foot, because the composition satisfactorily exhibits the effect of promoting blood circulation and good skin feeling can be sensed even on a body part with a thick horny layer.

In order to further improve the effect of promoting blood circulation and the effect of enhancing skin feeling at the time of bathing provided by the bath agent composition of the present invention, the bath agent composition of the present invention is one capable of adjusting the pH of bath water to preferably from 4 to 7, more preferably from 4.5 to 6.5.

In order to further improve the effect of promoting blood circulation provided by the bath agent composition of the present invention, the bath agent composition of the present invention is one capable of adjusting the concentration of carbon dioxide gas in bath water to preferably 30 ppm or more, more preferably 60 ppm or more.

In order to sufficiently enjoy the effect of promoting blood circulation and the effect of enhancing the skin feeling at the time of bathing provided by the bath agent composition of the present invention, the bathing temperature is preferably from 30°C to 45°C. In addition, it is desired that the bathing time be preferably 3 minutes or more, more preferably 5 minutes or more, even more preferably 10 minutes or more for each time of bathing.

With regard to the embodiments of the present invention described above, the following bath agent composition is further disclosed.
[1] A bath agent composition, comprising the following components (A), (B), and (C):
   (A) a total of 0.00001 mass% or more and 0.5 mass% or less of ginsenoside Rg3 and/or ginsenoside Rh2;
   (B) a carbonate comprising 30 mass% or more of a dialkali metal carbonate; and
   (C) an organic acid,
   wherein a mass ratio of ginsenosides Rb2, Rb1, Rc and Rd to ginsenosides Rg3 and Rh2 ((Rb2+Rb1+Rc+Rd)/(Rg3+Rh2)) is 0 or more and 30 or less.
[2] The bath agent composition according to the above-mentioned item [1], wherein the content of the component (A) is preferably 0.00002 mass% or more and is preferably 0.3 mass% or less in total.
[3] The bath agent composition according to the above-mentioned item [1] or [2], wherein the mass ratio ((Rb2+Rb1+Rc+Rd)/(Rg3+Rh2)) is preferably 20 or less, more preferably 10 or less, more preferably 7 or less, more preferably 3 or less, even more preferably 1 or less.
[4] The bath agent composition according to any one of the above-mentioned items [1] to [3], wherein the content of the dialkali metal carbonate in the component (B) is preferably 40 mass% or more, more preferably 50 mass% or more, and is preferably 100 mass% or less, more preferably 99 mass% or less, even more preferably 95 mass% or less.
[5] The bath agent composition according to any one of the above-mentioned items [1] to [4], wherein the dialkali metal carbonate comprises preferably one kind or two kinds selected from sodium carbonate and potassium carbonate, more preferably sodium carbonate.
[6] The bath agent composition according to any one of the above-mentioned items [1] to [5], wherein a carbonate other than the dialkali metal carbonate in the component (B) comprises preferably one kind or more kinds selected from a monoalkali metal carbonate and a carbonate of a divalent or higher-valent metal, more preferably a monoalkali metal carbonate, even more preferably one kind or more kinds selected from sodium hydrogen carbonate and potassium hydrogen carbonate.
[7] The bath agent composition according to any one of the above-mentioned items [1] to [6], wherein a content of the component (B) is preferably 20 mass% or more, more preferably 25 mass% or more, even more preferably 30 mass% or more, and is preferably 70 mass% or less, more preferably 65 mass% or less, even more preferably 60 mass% or less.
[8] The bath agent composition according to any one of the above-mentioned items [1] to [7], wherein a mass ratio of the ginsenosides Rg3 and Rh2 of the component (A) to the component (B), ((Rg3+Rh2)/(B)), is preferably 0.0005×10⁻³ or more, more preferably 0.0025×10⁻³ or more, even more preferably 0.02×10⁻³ or more, and is preferably 3×10⁻³ or less, more preferably 2.5×10⁻³ or less, even more preferably 2×10⁻³ or less.
[9] The bath agent composition according to any one of the above-mentioned items [1] to [8], wherein the component (C) comprises preferably one kind or more kinds selected from malic acid, tartaric acid, citric acid, maleic acid, succinic acid, phthalic acid, fumaric acid, glutaric acid, adipic acid, benzoic acid, salicylic acid and oxalic acid, more preferably one kind or more kinds selected from citric acid, malic acid, succinic acid and fumaric acid.
[10] The bath agent composition according to any one of the above-mentioned items [1] to [9], wherein a content of the component (C) is preferably 20 mass% or more, more preferably 25 mass% or more, even more preferably 30 mass% or more, and is preferably 70 mass% or less, more preferably 65 mass% or less, even more preferably 60 mass% or less.
[11] The bath agent composition according to any one of the above-mentioned items [1] to [10], further comprising a dye as a component (D), or free of the dye of the component (D).
[12] The bath agent composition according to the above-mentioned item [11], wherein the component (D) comprises preferably one kind or more kinds selected from a tar dye, a natural dye and a pigment, more preferably one kind or more kinds selected from tar dyes such as Blue No. 1, Red No. 106, Red No. 2, Yellow No. 4, Yellow No. 202(1) and Green No. 3, natural dyes such as chlorophyll, riboflavin and annatto, and pigments such as titanium oxide, zinc oxide, talc, kaolin, bentonite and titanated mica.
[13] The bath agent composition according to the above-mentioned item [11] or [12], wherein a content of the component (D) is preferably 0.0001 mass% or more and 5.0 mass% or less, more preferably 0.5 mass% or less, even more preferably 0.05 mass% or less, or the bath agent composition is preferably free of the dye of the component (D).
[14] The bath agent composition according to any one of the above-mentioned items [1] to [13], further comprising a water-soluble polymer as a component (E).
[15] The bath agent composition according to the above-mentioned item [14], wherein a content of the component (E) is preferably 0.5 mass% or more, more preferably 1 mass% or more, more preferably 3 mass% or more, even more preferably 5 mass% or more, and is preferably 30 mass% or less, more preferably 25 mass% or less.
[16] The bath agent composition according to the above-mentioned item [14] or [15], wherein the component (E) comprises preferably one kind or more kinds selected from a natural water-soluble polymer, a semisynthetic water-soluble polymer and a synthetic water-soluble polymer, more preferably one kind or two kinds selected from polyethylene glycol and dextrin, even more preferably polyethylene glycol.
[17] The bath agent composition according to any one of the above-mentioned items [14] to [16], wherein the component other than the component (B) is coated with the component (E).
[18] The bath agent composition according to the above-mentioned item [17], wherein the component other than the component (B) comprises the component (C).
[19] The bath agent composition according to any one of the above-mentioned items [1] to [18], further comprising an oily component.
[20] The bath agent composition according to the above-mentioned item [19], wherein a content of the oily component is preferably 10 mass% or less, more preferably 5 mass% or less.
[21] The bath agent composition according to any one of the above-mentioned items [1] to [20], wherein the bath agent composition has a pH of preferably from 4 to 7, more preferably from 4.5 to 6.5.
[22] The bath agent composition according to any one of the above-mentioned items [1] to [21], wherein the bath agent composition is a foot bath agent.
[23] Use of the bath agent composition of any one of the above-mentioned items [1] to [21], for foot bathing.
[24] Use of the bath agent composition of any one of the above-mentioned items [1] to [21], for producing a bath agent composition in which coloration and discoloration are suppressed.

### Examples

Now, the present invention is specifically described by way of Examples. It should be noted that the content of each component is represented in a mass% unit unless otherwise specifically stated in tables.

### [Production Example 1: Extraction of Extract]

850 mL of water was added to 100 g of the root of Asian ginseng (*Panax ginseng*) (made in China), and extraction was performed at 100°C for 30 hours, followed by filtration and then concentration, to yield a ginseng extract.

### [Production Example 2: Preparation of Granulated Product of Fumaric Acid Coated with PEG]

7.6 kg of fumaric acid and 0.4 kg of PEG 6000 were put into a Henschel mixer (FM20B type), heated and mixed until the temperature reached 70°C, and then cooled, to yield a granulated product. The mass ratio of fumaric acid to PEG, (fumaric acid/PEG), was 95/5.

### [Examples 1 to 16 and Comparative Examples 1 to 3]

Bath agents were each produced by mixing components according to a formulation shown in Tables 2 and 3 using the extract obtained in Production Example 1, and compression-molding the mixture into a foaming tablet having a weight of 45 g per tablet. It should be noted that the amounts of ginsenosides Rg3, Rh2, Rb2, Rb1, Rc and Rd in each bath agent were determined through analysis by high-performance liquid chromatography (HPLC) under the conditions shown in Table 1 below.

### HPLC Conditions

**[Table 1]**

| min | A | B |
|---|---|---|
| 0 | 65 | 35 |
| 2 | 65 | 35 |
| 6 | 50 | 50 |
| 11 | 50 | 50 |

Solvent: A: 0.01% TFA/H₂O, B: CH₃CN
Column: Poroshell 120 2.7 µm 3.0^{∗}75
Column temperature: 40°C
Detection: UV (210 nm)
Flow rate: 1 mL/min
Sample injection amount: 10 µL

The resultant bath agents were used and evaluated by the following methods. The results are shown in Tables 2 and 3.

### <<Evaluation of Promoting of Blood Circulation>>

Each bath agent having a weight of 45 g was put in 6 L of hot water at 40°C to prepare bath hot water having a carbon dioxide gas concentration of 1,000 ppm and a pH of 5.1, and five test subjects soaked their feet in the bath hot water for 15 minutes to perform foot bathing. Then, cutaneous blood flows (mL/min) were measured before the start of the foot bathing and immediately after the foot bathing. The blood flow of each subject before the start of the foot bathing was defined as 100, and the blood flow of each subject 15 minutes after the start of the foot bathing was represented as an index, followed by calculation of an average value of the results of the five subjects. It should be noted that the cutaneous blood flow was measured at the dorsum of the foot on which an electrode was placed using a laser Doppler blood-flowmeter (ALF21, manufactured by ADVANCE Co., Ltd.).

### <<Measurement of Color Difference>>

A color difference ΔE between each bath agent immediately after its production and the bath agent after being wrapped with an aluminum (7 µm) laminate film and preserved at 50°C for 1 day was measured with a spectrophotometer CM-700D (manufactured by KONICA MINOLTA, Inc.).

### <<Skin Feeling at the Time of Bathing>>

Three subjects each having healthy skin were allowed to bathe for 15 minutes in bath water obtained by dissolving each bath agent having a weight of 45 g in 6 L of bath water at 40°C, and sensorially evaluate skin feeling (moist, highly oily and smooth feeling can be sensed) at the time of bathing according to the following criteria. The average point was determined. A lower point indicates a more satisfactory result.
1: Very smooth
2: Smooth
3: No opinion
4: Less smooth
5: Not smooth

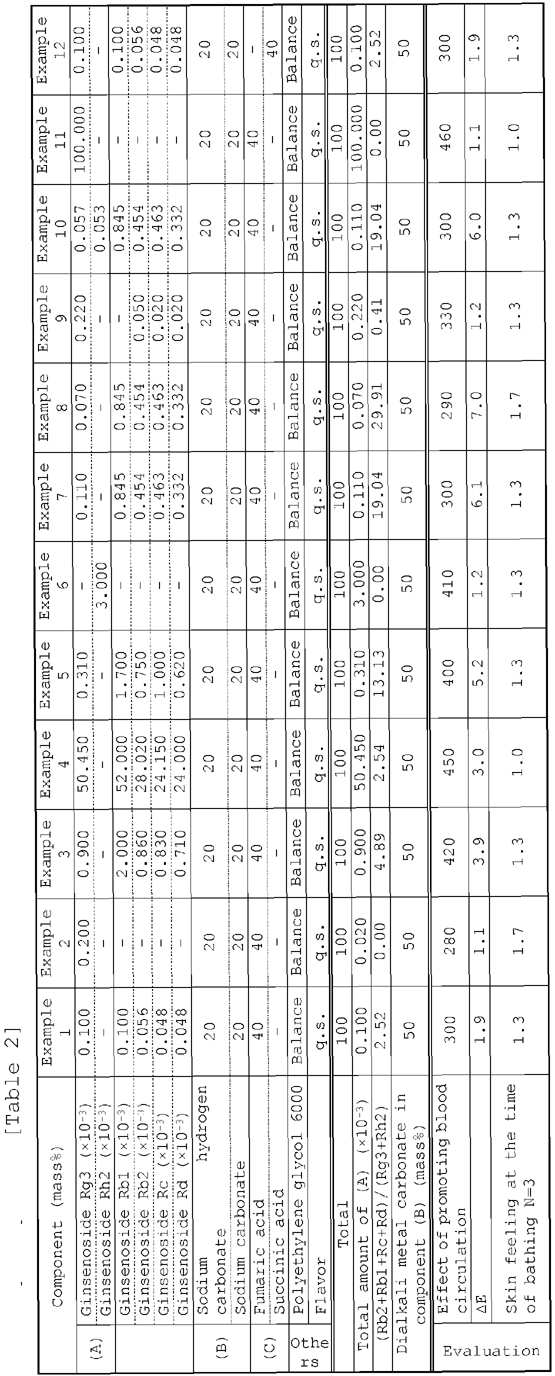

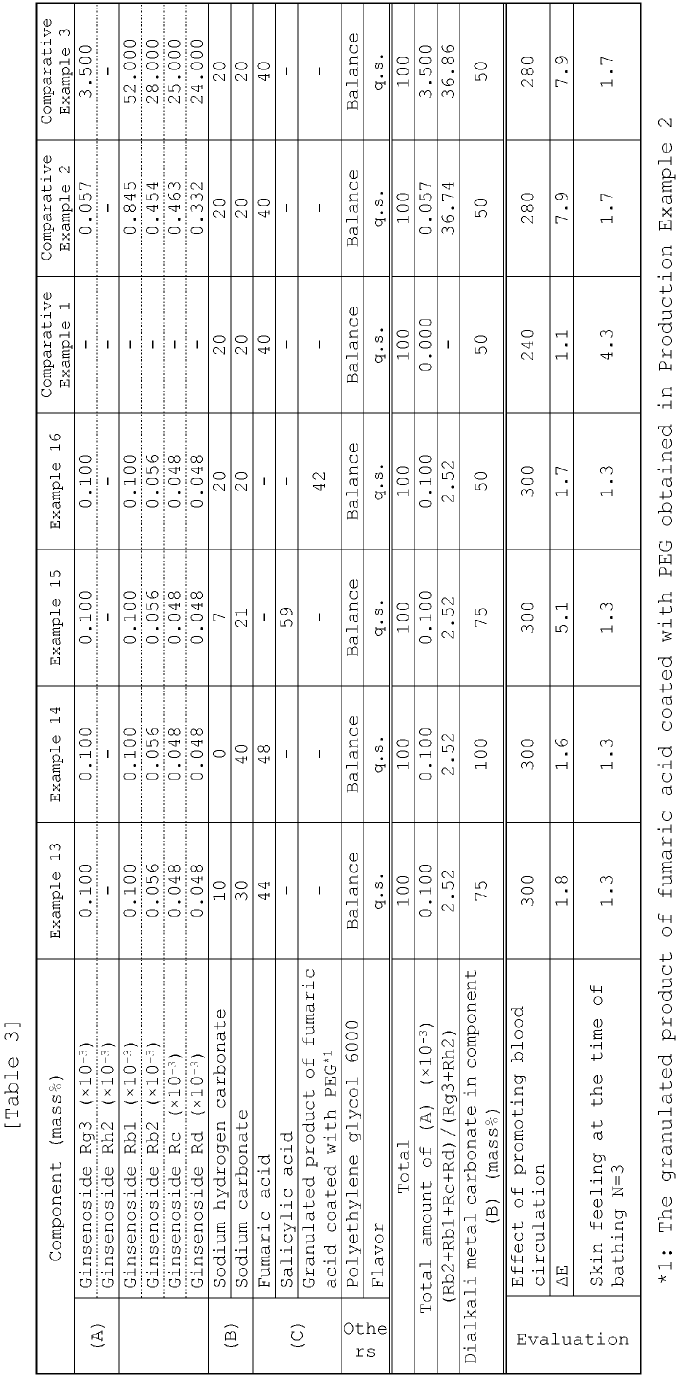

From the results shown in Tables 2 and 3, it is revealed that the bath agents of Examples 1 to 16 each exhibit an effect of suppressing coloration and discoloration and an effect of providing satisfactory skin feeling at the time of bathing in a balanced state, while satisfactorily exhibiting an effect of promoting blood circulation, as compared to the bath agent of Comparative Example 1 with no ginsenoside, and the bath agents of Comparative Examples 2 and 3 each having a mass ratio of ginsenosides Rg3 and Rh2 ((Rb2+Rb1+Rc+Rd)/(Rg3+Rh2)) of more than 30.

## Claims

1. A bath agent composition, comprising the following components (A), (B), and (C):
(A) a total of 0.00001 mass% or more and 0.5 mass% or less of ginsenoside Rg3 and/or ginsenoside Rh2;
(B) a carbonate comprising 30 mass% or more of a dialkali metal carbonate; and
(C) an organic acid,
wherein a mass ratio of ginsenosides Rb2, Rb1, Rc and Rd to ginsenosides Rg3 and Rh2 ((Rb2+Rb1+Rc+Rd)/(Rg3+Rh2)) is 0 or more and 30 or less.

2. The bath agent composition according to claim 1, which further comprises 0.001 mass% or more and 0.1 mass% or less of a dye as a component (D), or which is free of the dye of the component (D).

3. The bath agent composition according to claim 1 or 2, further comprising a water-soluble polymer as a component (E) .

4. The bath agent composition according to claim 3, wherein the component (C) is coated with the component (E).

5. The bath agent composition according to claim 3 or 4, wherein the component (E) comprises polyethylene glycol.

6. The bath agent composition according to any one of claims 1 to 5, wherein the mass ratio of ginsenosides Rb2, Rb1, Rc and Rd to ginsenosides Rg3 and Rh2 ((Rb2+Rb1+Rc+Rd)/(Rg3+Rh2)) is 0 or more and 10 or less.

7. The bath agent composition according to any one of claims 1 to 6, wherein the ginsenoside Rg3 and/or the ginsenoside Rh2 is obtained from *Panax ginseng.*

8. The bath agent composition according to any one of claims 1 to 7, wherein the component (C) comprises one kind or more kinds selected from citric acid, malic acid, succinic acid and fumaric acid.

9. The bath agent composition according to any one of claims 1 to 8, wherein the content of the dialkali metal carbonate in the component (B) is from 40 mass% to 100 mass%.

10. The bath agent composition according to any one of claims 1 to 9, wherein the dialkali metal carbonate is one kind or two kinds selected from sodium carbonate and potassium carbonate.

11. The bath agent composition according to any one of claims 1 to 10, wherein a mass ratio of ginsenosides Rg3 and Rh2 of the component (A) to the component (B), ((Rg3+Rh2)/(B)), is from 0.0005×10⁻³ to 3×10⁻³.

12. The bath agent composition according to any one of claims 1 to 11, further comprising an oily component.

13. The bath agent composition according to any one of claims 1 to 12, wherein the bath agent composition is a foot bath agent.

14. Use of the bath agent composition according to any one of claims 1 to 13 for whole-body bathing.

15. Use of the bath agent composition according to any one of claims 1 to 13 for foot bathing.

## Patentansprüche

1. Bademittelzusammensetzung, enthaltend die folgenden Komponenten (A), (B) und (C):
(A) insgesamt 0,00001 Masse-% oder mehr und 0,5 Masse-% oder weniger Ginsenosid Rg3 und/oder Ginsenosid Rh2,
(B) ein Carbonat, enthaltend 30 Masse-% oder mehr eines Dialkalimetallcarbonates und
(C) eine organische Säure,
worin ein Massenverhältnis der Ginsenoside Rb2, Rb1, Rc und Rd zu Ginsenosiden Rg3 und Rh2 ((Rb2+Rb1+Rc+Rd)/(Rg3+Rh2)) 0 oder mehr und 30 oder weniger ist.

2. Bademittelzusammensetzung gemäß Anspruch 1, weiterhin enthaltend 0,001 Masse-% oder mehr und 0,1 Masse-% oder weniger eines Farbstoffes als Komponente (D) oder die frei von Farbstoff der Komponente (D) ist.

3. Bademittelzusammensetzung gemäß Anspruch 1 oder 2, weiterhin enthaltend ein wasserlösliches Polymer als Komponente (E).

4. Bademittelzusammensetzung gemäß Anspruch 3, worin die Komponente (C) mit der Komponente (E) beschichtet ist.

5. Bademittelzusammensetzung gemäß Anspruch 3 oder 4, worin die Komponente (E) Polyethylenglykol enthält.

6. Bademittelzusammensetzung gemäß einem der Ansprüche 1 bis 5, worin das Massenverhältnis der Ginsenoside Rb2, Rb1, Rc und Rd zu Ginsenosiden Rg3 und Rh2 ((Rb2+Rb1+Rc+Rd)/(Rg3+Rh2)) 0 oder mehr und 10 oder weniger ist.

7. Bademittelzusammensetzung gemäß einem der Ansprüche 1 bis 6, worin das Ginsenosid Rg3 und/oder das Ginsenosid Rh2 von Panax giniseng erhalten ist.

8. Bademittelzusammensetzung gemäß einem der Ansprüche 1 bis 7, worin die Komponente (C) eine Art oder mehrere Arten enthält, ausgewählt aus Zitronensäure, Äpfelsäure, Succinsäure und Fumarsäure.

9. Bademittelzusammensetzung gemäß einem der Ansprüche 1 bis 8, worin der Gehalt des Dialkalimetallcarbonates in der Komponente (B) von 40 bis 100 Masse-% ist.

10. Bademittelzusammensetzung gemäß einem der Ansprüche 1 bis 9, worin das Dialkalimetallcarbonat eine Art oder zwei Arten ist, ausgewählt aus Natriumcarbonat und Kaliumcarbonat.

11. Bademittelzusammensetzung gemäß einem der Ansprüche 1 bis 10, worin ein Massenverhältnis von Ginsenosiden Rg3 und Rh2 der Komponente (A) zu der Komponente (B), ((Rg3+Rh2)/(B)), von 0,0005×10⁻³ bis 3×10⁻³ ist.

12. Bademittelzusammensetzung gemäß einem der Ansprüche 1 bis 11, weiterhin enthaltend eine ölige Komponente.

13. Bademittelzusammensetzung gemäß einem der Ansprüche 1 bis 12, worin die Bademittelzusammensetzung ein Fußbademittel ist.

14. Verwendung der Bademittelzusammensetzung gemäß einem der Ansprüche 1 bis 13 zum Baden des gesamten Körpers.

15. Verwendung der Bademittelzusammensetzung gemäß einem der Ansprüche 1 bis 13 zum Fußbaden.

## Revendications

1. Composition d'agent de bain, comprenant les composants (A), (B) et (C) suivants :
(A) un total de 0,00001 % en masse ou plus et de 0,5 % en masse ou moins de ginsénoside Rg3 et/ou de ginsénoside Rh2 ;
(B) un carbonate comprenant 30 % en masse ou plus d'un carbonate de métal dialcalin ; et
(C) un acide organique,
dans lequel un rapport massique de ginsénosides Rb2, Rb1, Rc et Rd aux ginsénosides Rg3 et Rh2 ((Rb2+Rb1+Rc+Rd) / (Rg3+Rh2)) est de 0 ou plus et de 30 ou moins.

2. Composition d'agent de bain selon la revendication 1, qui comprend en outre 0,001 % en masse ou plus et 0,1 % en masse ou moins d'un colorant comme composant (D), ou qui est exempt du colorant du composant (D).

3. Composition d'agent de bain selon la revendication 1 ou 2, comprenant en outre un polymère soluble dans l'eau comme composant (E).

4. Composition d'agent de bain selon la revendication 3, dans laquelle le composant (C) est revêtu du composant (E).

5. Composition d'agent de bain selon la revendication 3 ou 4, dans lequel le composant (E) contient du polyéthylène glycol.

6. Composition d'agent de bain selon l'une quelconque des revendications 1 à 5, dans laquelle le rapport massique des ginsénosides Rb2, Rb1, Rc et Rd aux ginsénosides Rg3 et Rh2 ((Rb2+Rb1+Rc+Rd) / (Rg3+Rh2)) est de 0 ou plus et de 10 ou moins.

7. Composition d'agent de bain selon l'une quelconque des revendications 1 à 6, dans laquelle le ginsénoside Rg3 et/ou le ginsénoside Rh2 est ou sont obtenus à partir de *Panax ginseng.*

8. Composition d'agent de bain selon l'une quelconque des revendications 1 à 7, dans laquelle le composant (C) comprend un ou plusieurs types choisis parmi l'acide citrique, l'acide malique, l'acide succinique et l'acide fumarique.

9. Composition d'agent de bain selon l'une quelconque des revendications 1 à 8, dans laquelle la teneur en carbonate de métal dialcalin dans le composant (B) est de 40 % en masse à 100 % en masse.

10. Composition d'agent de bain selon l'une quelconque des revendications 1 à 9, dans laquelle le carbonate de métal dialcalin est d'un ou deux types choisis parmi le carbonate de sodium et le carbonate de potassium.

11. Composition d'agent de bain selon l'une quelconque des revendications 1 à 10, dans laquelle un rapport massique des ginsénosides Rg3 et Rh2 du composant (A) au composant (B) ((Rg3+Rh2) / (B)) est de 0,0005 x 10⁻³ à 3 x 10⁻³.

12. Composition d'agent de bain selon l'une quelconque des revendications 1 à 11, comprenant en outre un composant huileux.

13. Composition d'agent de bain selon l'une quelconque des revendications 1 à 12, dans laquelle la composition d'agent de bain est un agent de bain de pieds.

14. Utilisation de la composition d'agent de bain selon l'une quelconque des revendications 1 à 13 pour bain du corps entier.

15. Utilisation de la composition d'agent de bain selon l'une quelconque des revendications 1 à 13 pour bain de pieds.
